# EUROPEAN PATENT APPLICATION

(11) **EP 4 745 115 A1**
(43) Date of publication of application: **20.05.2026**
(21) Application number: 24891066.3
(22) Date of filing: 05.09.2024
(51) Int. Cl.: C07C 29/151, C01B 3/02, C01B 32/50, C07C 31/04, C07B 61/00

(54) **METHOD FOR SYNTHESIZING METHANOL**

(30) Priority: 16.11.2023 JP 2023195399
(71) Applicant: JFE Steel Corporation, Tokyo, 100-0011 (JP)
(72) Inventor: YOSHIKAWA, Kohei, Tokyo 100-0011 (JP); SHIGAKI, Nobuyuki, Tokyo 100-0011 (JP); NISHIKAWA, Yuta, Tokyo 100-0011 (JP); OKIDA, Tomoyuki, Tokyo 100-0011 (JP)
(74) Representative: Hoffmann Eitle
(86) International application number: PCT/JP2024/031947
(87) International publication number: WO 2025/105024

(57) **Abstract**

A method of more stably synthesizing methanol from a gas containing carbon dioxide and sulfur compounds, such as blast furnace gas. The method includes: a water addition process of adding water to a feed gas G01; a hydrolysis process of hydrolyzing carbonyl sulfide contained in obtained hydrated feed gas into hydrogen sulfide and carbon dioxide; a water-gas shift reaction process of converting carbon monoxide and water contained in obtained hydrolyzed gas into carbon dioxide and hydrogen; a carbon dioxide gas separation process of separating obtained shift reaction gas into carbon dioxide gas and gases of components other than carbon dioxide; a desulfurization process of removing at least hydrogen sulfide from hydrogen sulfide and carbonyl sulfide contained in the carbon dioxide gas to obtain a high-concentration carbon dioxide gas; a hydrogenation process of adding hydrogen gas to the high-concentration carbon dioxide gas; and a methanol synthesis process of reacting carbon dioxide and hydrogen contained in obtained methanol feed gas.

## Description

### TECHNICAL FIELD

The present disclosure relates to methods of synthesizing methanol, and more particularly to a method of synthesizing methanol from a gas containing carbon dioxide and sulfur compounds such as hydrogen sulfide and carbonyl sulfide.

### BACKGROUND

In recent years, there has been demand to reduce carbon dioxide (CO₂) emissions in order to help prevent global warming. Steelworks emit large amounts of by-product gases, including blast furnace gas as a by-product of blast furnaces, coke oven gas from coke ovens, and converter gas from converters. In particular, blast furnace gas, which is emitted in particularly large amounts, contains a large amount of CO₂. By-product gases such as blast furnace gas are used as energy sources within steelworks, but the CO₂ contained in blast furnace gas is simply emitted. Therefore, there is an urgent need to decrease CO₂ emissions.

Against this background, in Patent Literature (PTL) 1, a method is proposed for synthesizing methanol from a feed gas containing CO₂ and hydrogen (H₂) using a catalyst as a method for effectively utilizing CO₂.

Further, in PTL 2, a method is proposed of improving the efficiency of methanol synthesis by separating water (H₂O) using a membrane reactor having a dehydration membrane, and further obtaining H₂ by a water-gas shift reaction using the separated H₂O.

### CITATION LIST

### Patent Literature

PTL 1: JP 7049075 B2
PTL 2: JP 2020-132439 A

### SUMMARY

### (Technical Problem)

In addition to the main components CO₂, carbon monoxide (CO), and H₂, blast furnace gas also contains sulfur compounds such as hydrogen sulfide (H₂S) and carbonyl sulfide (COS), which are derived from the sulfur in the iron ore and coke used as raw materials. H₂S and COS are known to cause deterioration of copper catalysts used in methanol synthesis.

In the method proposed in PTL 1, when blast furnace gas is used as a feed gas, H₂S and COS flow through the methanol synthesis catalyst, which causes a problem of deteriorating the catalyst.

Further, PTL 2 describes that H₂ obtained by the water-gas shift reaction is separated by pressure swing adsorption, but does not describe the use of CO₂ produced as a by-product. When CO₂ is separated by pressure swing adsorption and used for methanol synthesis, the H₂S and COS are concentrated on the separated CO₂ side, which also causes the problem of deteriorating the methanol synthesis catalyst.

Further, PTL 2 describes that the water-gas shift reaction is carried out using blast furnace gas as a sweep gas for H₂O, but does not describe the type of catalyst used. However, copper catalysts typically used in the water-gas shift reaction have the problem of being deteriorated by H₂S and COS.

As described above, the methods described in PTL 1 and 2 make it difficult to synthesize methanol stably.

In view of the above problems, it would be helpful to provide a method of more stably synthesizing methanol from a gas containing carbon dioxide and sulfur compounds, such as blast furnace gas.

### (Solution to Problem)

In order to solve the technical problems described above, the following are provided:
[1] A method of synthesizing methanol from hydrogen gas and a feed gas containing carbon dioxide, carbon monoxide, hydrogen sulfide, and carbonyl sulfide, the method comprising:
   a water addition process of adding water to the feed gas to obtain a hydrated feed gas;
   a hydrolysis process of hydrolyzing the carbonyl sulfide contained in the hydrated feed gas into hydrogen sulfide and carbon dioxide to obtain a hydrolyzed gas;
   a water-gas shift reaction process of subjecting the hydrolyzed gas to a water-gas shift reaction to convert the carbon monoxide and the water contained in the hydrolyzed gas into carbon dioxide and hydrogen to obtain a shift reaction gas;
   a carbon dioxide gas separation process of separating the shift reaction gas into carbon dioxide gas and gases of components other than carbon dioxide;
   a desulfurization process of removing at least hydrogen sulfide from hydrogen sulfide and carbonyl sulfide contained in the carbon dioxide gas to obtain a high-concentration carbon dioxide gas;
   a hydrogenation process of adding hydrogen gas to the high-concentration carbon dioxide gas to obtain a methanol feed gas; and
   a methanol synthesis process of reacting carbon dioxide and hydrogen contained in the methanol feed gas to produce methanol.
[2] The method of synthesizing methanol according to [1], wherein water added to the feed gas in the water addition process is water produced as a by-product in the methanol synthesis process.
[3] The method of synthesizing methanol according to [2], wherein the methanol synthesis process includes a membrane separation process of separating the water produced as a by-product of the reaction of the carbon dioxide and the hydrogen by passing the water through a membrane, and the water separated in the membrane separation process is used as water added to the feed gas in the water addition process.
[4] The method of synthesizing methanol according to [3], wherein the membrane separation process is carried out in the same vessel as the methanol synthesis process.
[5] The method of synthesizing methanol according to [3] or [4], wherein the membrane separation process includes a sweep process of passing the feed gas through one side of the membrane and sweeping water that has permeated the membrane with a sweep gas, with the feed gas being used as the sweep gas, and the feed gas to which water that has permeated the membrane is added being the hydrated feed gas.
[6] The method of synthesizing methanol according to any one of [1] to [5], wherein a method used in the desulfurization process is a dry desulfurization method using a dry desulfurizing agent containing an iron compound selected from iron oxide and iron hydroxide.
[7] The method of synthesizing methanol according to [6], wherein part or all of the iron compound is derived from a precipitate generated in water treatment at a steelworks.
[8] The method of synthesizing methanol according to any one of [1] to [5], wherein a method used in the desulfurization process is a dry desulfurization method using a dry desulfurizing agent containing at least one compound of an element selected from zinc and copper.
[9] The method of synthesizing methanol according to any one of [1] to [8], further comprising a heat exchange process of exchanging heat between the shift reaction gas and the hydrolyzed gas.
[10] The method of synthesizing methanol according to any one of [5] to [9], further comprising a water washing process of bringing the feed gas into contact with liquid water and washing, before the sweep process.
[11] The method of synthesizing methanol according to any one of [1] to [10], wherein a catalyst used in the hydrolysis process is a catalyst containing an oxide of Al.
[12] The method of synthesizing methanol according to any one of [1] to [11], wherein a catalyst used in the water-gas shift reaction process is a catalyst containing a sulfide of at least one element selected from Co, Ni, and Mo.
[13] The method of synthesizing methanol according to any one of [1] to [12], wherein a catalyst used in the water-gas shift reaction process is a catalyst containing an oxide of at least one element selected from Fe and Cr.
[14] The method of synthesizing methanol according to any one of [1] to [13], further comprising a hydrogen gas separation process of separating hydrogen gas from gas of components other than the carbon dioxide separated in the carbon dioxide gas separation process.
[15] The method of synthesizing methanol according to any one of [1] to [14], wherein the feed gas is an exhaust gas discharged from a reduction furnace.
[16] The method of synthesizing methanol according to [15], wherein the reduction furnace is a blast furnace in a steelworks, and the feed gas is blast furnace gas.

### (Advantageous Effect)

According to the present disclosure, methanol can be synthesized more stably from a gas containing carbon dioxide and sulfur compounds, such as blast furnace gas.

### BRIEF DESCRIPTION OF THE DRAWINGS

In the accompanying drawings:
FIG. 1 is a flow chart of a preferred example of a method of synthesizing methanol according to the present disclosure; and
FIG. 2 is a flow chart of another preferred example of the method of synthesizing methanol according to the present disclosure.

### DETAILED DESCRIPTION

An embodiment of the present disclosure is described below with reference to the drawings. A method of synthesizing methanol according to the present disclosure is a method of synthesizing methanol from hydrogen gas and a feed gas containing carbon dioxide, carbon monoxide, hydrogen sulfide, and carbonyl sulfide, and includes: a water addition process of adding water to the feed gas to obtain a hydrated feed gas; a hydrolysis process of hydrolyzing the carbonyl sulfide contained in the hydrated feed gas into hydrogen sulfide and carbon dioxide to obtain a hydrolyzed gas; a water-gas shift reaction process of subjecting the hydrolyzed gas to a water-gas shift reaction to convert the carbon monoxide and the water contained in the hydrolyzed gas into carbon dioxide and hydrogen to obtain a shift reaction gas; a carbon dioxide gas separation process of separating the shift reaction gas into carbon dioxide gas and gases of components other than carbon dioxide; a desulfurization process of removing at least hydrogen sulfide from hydrogen sulfide and carbonyl sulfide contained in the carbon dioxide gas to obtain a high-concentration carbon dioxide gas; a hydrogenation process of adding hydrogen gas to the high-concentration carbon dioxide gas to obtain a methanol feed gas; and a methanol synthesis process of reacting carbon dioxide and hydrogen contained in the methanol feed gas to produce methanol.

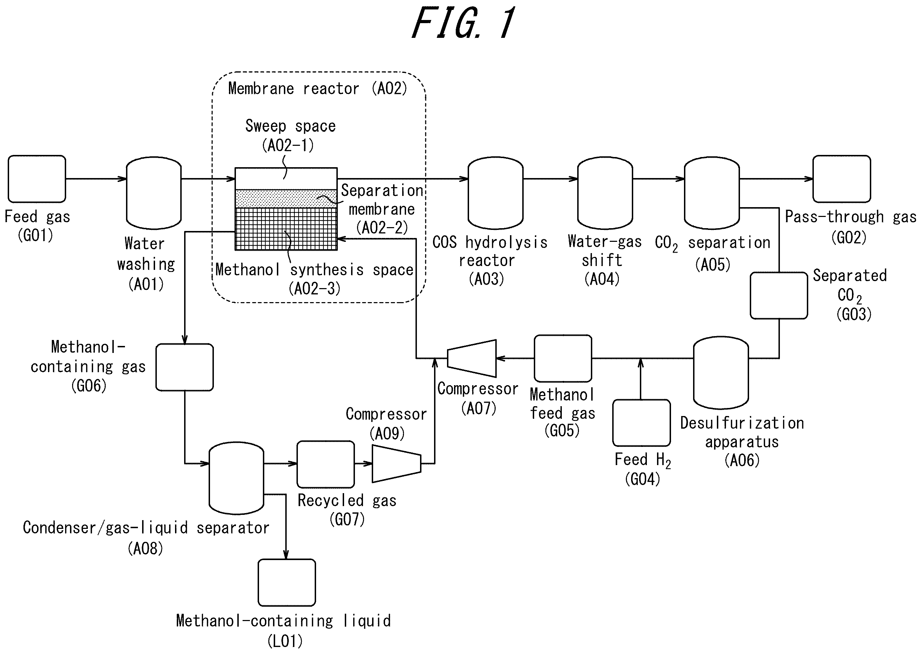

FIG. 1 is a flow chart of a preferred example of the method of synthesizing methanol according to the present disclosure. The present disclosure is described in detail below, following the flow chart illustrated in FIG. 1. The method of synthesizing methanol according to the present disclosure is a method of synthesizing methanol from H₂ gas and a feed gas (G01) containing CO₂, CO, H₂S, and COS.

As such a feed gas (G01), an exhaust gas discharged from a reduction furnace in a steelworks can be suitably used. Examples of the reduction furnace include a blast furnace, a coke oven, and a gasifier, and examples of the feed gas (G01) that can be used include blast furnace gas, coke oven gas, and gasifier gas. Among these, blast furnace gas is preferably used as the feed gas (G01), which allows stable synthesis of methanol. In the following description, an example is described in which the feed gas G01 contains N₂ as a component, which is often contained in exhaust gas discharged from reduction furnaces.

The feed gas (G01) may contain H₂ gas, and depending on the content of the H₂ gas contained in the feed gas (G01), additional H₂ gas required for the synthesis of methanol can be prepared separately to synthesize methanol.

First, preferably, the feed gas (G01) is supplied to a water washer (A01) and the feed gas (G01) is brought into contact with liquid phase H₂O for washing (water washing process). The water washing process can adjust the H₂O contained in the feed gas (G01) and decrease dust and the like in the feed gas (G01). A temperature of the H₂O used in the water washing process may be set based on the ambient temperature and the like, and is preferably 0 °C or higher. The temperature of the H₂O is preferably 100 °C or lower. The temperature of the H₂O is more preferably 10 °C or higher. The temperature of the H₂O is more preferably 40 °C or lower.

Next, the water-washed feed gas (G01) is passed through a sweep space (A02-1) in a membrane reactor (A02), and H₂O is added to the feed gas (G01) to obtain a hydrated feed gas (water addition process).

The membrane reactor (A02) includes the sweep space (A02-1), a separation membrane (A02-2), and a methanol synthesis space (A02-3). As described later, methanol is synthesized in the methanol synthesis space (A03-3), and H₂O produced as a by-product in the synthesis permeates the separation membrane (A02-2) and flows into the sweep space (A02-1). Therefore, it is preferable to pass the feed gas (G01) as a sweep gas through the sweep space (A02-1) to sweep out the H₂O that has permeated the separation membrane (A02-2) (sweep process). This allows H₂O to be efficiently added to the feed gas (G01) to produce a hydrated feed gas, thereby decreasing the load of separately adding H₂O to the feed gas (G01).

The temperature of the hydrated feed gas discharged from the sweep space (A02-1) may vary depending on the conditions for the subsequent methanol synthesis and the properties of the separation membrane (A02-2). However, the temperature is set to be an appropriate temperature for subsequent COS hydrolysis, preferably 100 °C or higher. The temperature is preferably 250 °C or lower. The temperature is more preferably 150 °C or higher. The temperature is more preferably 200 °C or lower.

Next, the hydrated feed gas is introduced into a COS hydrolysis reactor (A03), where the COS contained in the hydrated feed gas is hydrolyzed into H₂S and CO₂ to produce a hydrolyzed gas (hydrolysis process).

As a catalyst used in the hydrolysis process, a catalyst containing an oxide of aluminum (Al) is preferably used. This improves the efficiency of hydrolysis, allowing efficient COS hydrolysis to be carried out, thereby increasing desulfurization efficiency in the desulfurization process described below. More preferably, the catalyst further contains potassium (K) in addition to the oxide of Al.

The reaction temperature for hydrolysis may be set based on activity and chemical equilibrium of the catalyst, and as described above, is preferably 100 °C or higher. The reaction temperature is preferably 250 °C or lower. The reaction temperature is more preferably 150 °C or higher. The reaction temperature is more preferably 200 °C or lower.

The COS hydrolysis reactor (A03) is preferably upstream of a water-gas shift reactor (A04) described below, and the COS hydrolysis process is preferably carried out prior to a water-gas shift reaction process described below. This is because the water-gas shift reaction is an exothermic reaction, and a temperature downstream of the water-gas shift reactor (A04) rises to a temperature higher than that suitable for COS hydrolysis.

Further, when a gas having a high CO concentration (for example, about 20 vol%) such as blast furnace gas is used as the feed gas (G01), H₂O is consumed during the water-gas shift reaction process, and therefore the H₂O concentration decreases downstream of the water-gas shift reactor (A04), which may result in a gas composition that is unsuitable for COS hydrolysis. Although COS hydrolysis is also an exothermic reaction that consumes H₂O, the COS concentration in blast furnace gas (for example, 100 ppm or less) is much lower than the CO concentration. Therefore, the change in gas temperature and H₂O concentration caused by the COS hydrolysis reactor (A03) is considered to be minimal, and the influence on the water-gas shift reactor (A04) is also considered to be minimal.

In the hydrolysis process, some COS remains due to chemical equilibrium, but the remaining COS can be removed in the subsequent desulfurization process.

The hydrolyzed gas is then introduced into the water-gas shift reactor (A04), where the CO and H₂O contained in the hydrolyzed gas are subjected to a water-gas shift reaction to convert into CO₂ and H₂, thereby producing a shift reaction gas (water-gas shift reaction process).

The catalyst used in the water-gas shift reaction process may be a catalyst containing at least one sulfide of an element selected from cobalt (Co), nickel (Ni), and molybdenum (Mo). This allows the water-gas shift reaction and the COS hydrolysis reaction to proceed efficiently even when a sulfur compound is present in the stream, thereby increasing the desulfurization efficiency. As a result, deterioration of catalytic performance can be suppressed, and methanol can be synthesized stably.

Further, the catalyst used in the water-gas shift reaction process may contain at least one oxide of an element selected from iron (Fe) and chromium (Cr). This allows the shift reaction to proceed efficiently and stably even when a sulfur compound is present in the stream.

The reaction temperature of the water-gas shift reaction may be set based on the activity and chemical equilibrium of the catalyst, and with inlet gas temperature as an example, is preferably 200 °C or higher and 400 °C or lower. The inlet gas temperature is more preferably 200 °C or higher and 250 °C or lower.

Next, the shift reaction gas is introduced into a CO₂ separation device (A05), and the shift reaction gas is separated into CO₂ gas (separated CO₂ gas (G03)) and a pass-through gas (G02) that is a gas of components other than CO₂ (carbon dioxide gas separation process).

In the CO₂ separation device (A05), the gas is separated into the separated CO₂ gas (G03) containing mainly CO₂ and also containing one or both of COS and H₂S, and a gas (G02) of components other than CO₂, specifically, the pass-through gas (G02) containing mainly N₂, CO, and H₂.

The CO₂ separation method may be selected from various methods such as chemical absorption using amines, physical absorption using a physical absorption liquid, pressure swing adsorption using an adsorbent, and membrane separation using a separation membrane. For example, for a gas having a high CO₂ partial pressure such as in a reduction furnace, a pressure swing adsorption method using zeolite or the like as a CO₂ adsorbent is preferred.

When a pressure swing adsorption method is used, the concentration of H₂O contained in the shift reaction gas is preferably decreased in advance. Methods for decreasing the H₂O concentration include, for example, cooling and condensation using a condenser, and a desiccant method using zeolite, alumina, silica, or the like as an H₂O adsorbent. An appropriate range of the dew point of dehumidified shift reaction gas varies depending on the CO₂ adsorbent. For example, when zeolite is used as the CO₂ adsorbent, the dew point is preferably 0 °C or lower. The dew point is more preferably -20 °C or lower.

Subsequently, the separated CO₂ gas (G03) is introduced into a desulfurization apparatus (A06) to remove at least H₂S from H₂S and COS contained in the separated CO₂ gas (G03) to obtain a high-concentration CO₂ gas (desulfurization process).

Examples of the desulfurization apparatus (A06) include apparatus based on a scrubber using a chemical solution, a chemical absorption method using an amine, a physical absorption method using a physical absorption solution, and a dry desulfurization method using a desulfurizing agent. When the sulfur concentration of the separated CO₂ gas (G03) is decreased to 0.1 ppm or less, dry desulfurization is preferably used. Examples of dry desulfurization methods include a multi-stage desulfurization method in which a reactor loaded with a dry desulfurizing agent containing an iron compound selected from iron oxide or iron hydroxide is a first stage, and a reactor loaded with a dry desulfurizing agent containing at least one compound selected from zinc (Zn) and copper (Cu) such as copper oxide, is a second stage. In such a case, H₂S is removed in the first reactor and COS is removed in the second reactor. The temperature in these reactors of the desulfurization apparatus (A06) is, for example, preferably 10 °C or higher and 50 °C or lower in the case of a reactor loaded with a desulfurizing agent containing Fe, and 10 °C or higher and 400 °C or lower in the case of a reactor loaded with a dry desulfurizing agent containing at least one compound of an element selected from Zn and Cu such as copper oxide.

Thereafter, feed H₂ gas (G04) is added to the high concentration CO₂ to produce a methanol feed gas (G05) (hydrogenation process).

Examples of methods to obtain the feed H₂ gas (G04) include vaporizing liquefied hydrogen, electrolyzing H₂O, and separation from H₂ containing gas.

Next, the methanol feed gas (G05) is compressed by a compressor (A07) and then passed through the methanol synthesis space (A02-3) loaded with a methanol synthesis catalyst inside the membrane reactor (A02). Here, CO₂ and H₂ contained in the methanol feed gas (G05) are reacted with each other to synthesize methanol according to the following reaction equation (1):

CO₂ + 3H₂ → CH₃OH + H₂O (1)

The pressure applied in the compressor (A07) is preferably 1.0 MPa or more. The pressure is more preferably 3.0 MPa or more. In order to prevent heating and a decrease in compression efficiency during compression, the feed gas (G05) is preferably compressed using a multi-stage compressor (A07).

A part of the H₂O produced in the methanol synthesis space (A02-3) permeates the separation membrane (A02-2) and flows into the sweep space (A02-1), and is swept by the feed gas (G01) as described above.

The sweep space (A02-1), the separation membrane (A02-2), and the methanol synthesis space (A02-3) in the membrane reactor (A02) are configured in the same vessel. This makes it possible to change the chemical equilibrium of methanol synthesis in favor of methanol production by separating H₂O through membrane separation, thereby improving the efficiency of methanol synthesis.

The catalyst to be loaded in the methanol synthesis space (A02-3) may be a catalyst containing Cu or Zn. Further, the reaction temperature for methanol synthesis may be set based on the activity of the catalyst, gas pressure, chemical equilibrium, and the like, and, for example, is preferably 200 °C or higher and 250 °C or lower. The reaction temperature is more preferably 200 °C or higher and 230 °C or lower.

Meanwhile, the methanol-containing gas (G06) discharged from the methanol synthesis space (A02-3) is introduced into a condenser/gas-liquid separator (A08) where it is cooled and separated into a methanol-containing liquid (L01) and a recycled gas (G07). The separated recycled gas (G07) is introduced into a compressor (A09) to be pressurized, and then passed through the methanol synthesis space (A02-3) again.

The temperature at which methanol is condensed from the methanol-containing gas (G06) after synthesis in the condenser/gas-liquid separator (A08) is preferably a low temperature equal to or higher than the solidification point of methanol, preferably 50 °C or lower. The temperature is more preferably 30 °C or lower. The condensed methanol-containing liquid (L01) and the recycled gas (G07) are separated by gas-liquid separation.

Thus, according to the flowchart illustrated in FIG. 1, by synthesizing methanol while removing H₂O using the membrane reactor (A02), it is possible to alleviate equilibrium constraints and increase the efficiency of methanol synthesis. Further, the H₂O separated by the separation membrane (A02-2) allows the COS hydrolysis and the water-gas shift reaction to proceed satisfactorily. Further, in the sweep process, the hydrated feed gas is heated by the heat generated in the methanol synthesis process and raised to a temperature appropriate for COS hydrolysis, thereby decreasing the energy consumption required to increase the temperature of the hydrated feed gas.

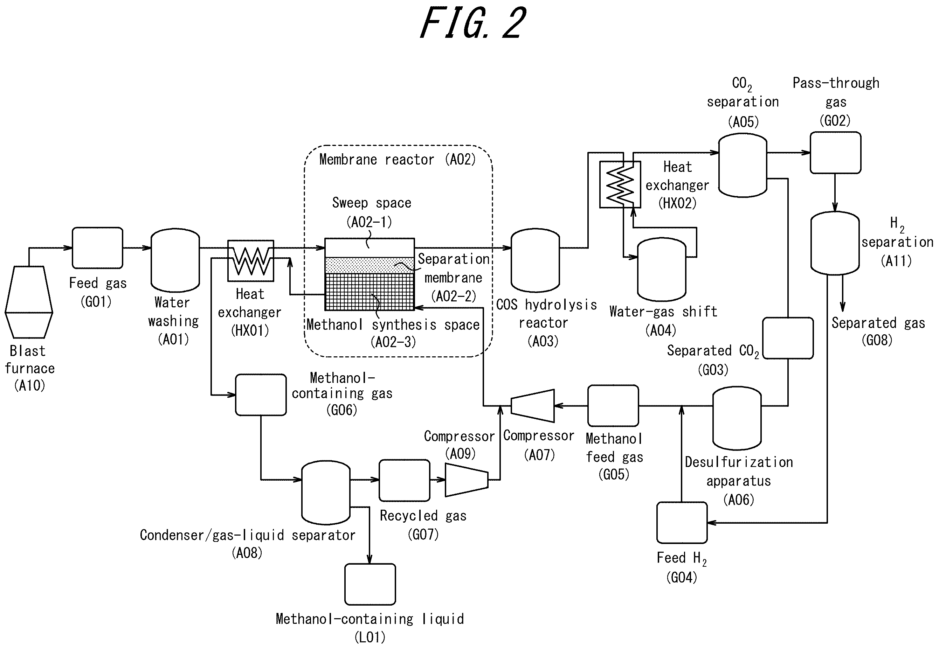

FIG. 2 illustrates a flowchart of another preferred example of the method of producing methanol according to the present disclosure. The flowchart illustrated in FIG. 2 differs from the flowchart illustrated in FIG. 1 in that, in addition: (A) blast furnace gas discharged from a blast furnace (A10) of a steelworks is used as the feed gas (G01); (B) a heat exchange process (first heat exchange process) by a heat exchanger HX1 is further provided in which heat is exchanged between the feed gas (G01) after the water washing process in the water washer (A01) and the methanol-containing gas (G06) discharged from the methanol synthesis space (A02-3); (C) a heat exchange process (second heat exchange process) by a heat exchanger HX2 is provided in which heat is exchanged between the hydrolyzed gas discharged from the COS hydrolysis reactor (A03) and the shift reaction gas discharged from the water-gas shift reactor (A04); and (D) a hydrogen gas separation process is further provided in which H₂ gas is separated from the pass-through gas (G02) discharged from the CO₂ separation device (A05) using a H₂ separation device (A11), and the hydrogen gas is used as part of the feed H₂ gas (G04).

Regarding the difference (B), heat exchange between the feed gas (G01) after the water washing process in the water washer (A01) and the methanol-containing gas (G06) discharged from the methanol synthesis space (A02-3) increases the temperature of the feed gas (G01) flowing through the sweep space (A02-1), thereby making it possible to suppress a decrease in the activity of the methanol synthesis catalyst. The temperature of the feed gas (G01) is, for example, preferably 100 °C or higher. The temperature is more preferably 150 °C or higher.

Regarding the difference (C), the temperature of the hydrolyzed gas can be set to a temperature appropriate for the hydrolysis reaction by utilizing the heat generated in the water-gas shift reaction through heat exchange between the hydrolyzed gas discharged from the COS hydrolysis reactor (A03) and the shift reaction gas discharged from the water-gas shift reactor (A04). The temperature of the hydrolyzed gas discharged from the COS hydrolysis reactor (A03) after heat exchange may be set based on the activity of the catalyst and chemical equilibrium, and is, for example, preferably 200 °C or higher. The temperature is preferably 300 °C or lower. The temperature is more preferably 220 °C or higher. The temperature is more preferably 260 °C or lower.

Regarding the difference (D), in the H₂ separation device (A11), the gas is separated into H₂ gas containing mainly H₂ and separated gas (G08) containing mainly N₂ and CO. The H₂ separation method may be selected from various methods such as pressure swing adsorption using an adsorbent and membrane separation using a separation membrane. By using the H₂ gas separated by this device as the feed H₂ gas (G04), the cost of the feed H₂ gas can be decreased.

### INDUSTRIAL APPLICABILITY

The present disclosure is useful in the steel industry due to enabling more stable synthesis of valuable materials such as methanol from gases containing carbon dioxide and sulfur compounds, such as blast furnace gas.

### REFERENCE SIGNS LIST

- A01: water washer
- A02: membrane reactor
- A02-1: sweep space
- A02-2: separation membrane
- A02-3: methanol synthesis space
- A03: COS hydrolysis reactor
- A04: water-gas shift reactor
- A05: CO₂ separation device
- A06: desulfurization apparatus
- A07, A09: compressor
- A08: condenser/gas-liquid separator
- A10: blast furnace
- A11: H₂ separation device
- G01: feed gas
- G02: pass-through gas
- G03: separated CO₂ gas
- G04: feed H₂ gas
- G05: methanol feed gas
- G06: methanol-containing gas
- G07: recycled gas
- G08: separated gas
- HX1, HX2: heat exchanger
- L01: methanol-containing liquid

## Claims

1. A method of synthesizing methanol from hydrogen gas and a feed gas containing carbon dioxide, carbon monoxide, hydrogen sulfide, and carbonyl sulfide, the method comprising:
a water addition process of adding water to the feed gas to obtain a hydrated feed gas;
a hydrolysis process of hydrolyzing the carbonyl sulfide contained in the hydrated feed gas into hydrogen sulfide and carbon dioxide to obtain a hydrolyzed gas;
a water-gas shift reaction process of subjecting the hydrolyzed gas to a water-gas shift reaction to convert the carbon monoxide and the water contained in the hydrolyzed gas into carbon dioxide and hydrogen to obtain a shift reaction gas;
a carbon dioxide gas separation process of separating the shift reaction gas into carbon dioxide gas and gases of components other than carbon dioxide;
a desulfurization process of removing at least hydrogen sulfide from hydrogen sulfide and carbonyl sulfide contained in the carbon dioxide gas to obtain a high-concentration carbon dioxide gas;
a hydrogenation process of adding hydrogen gas to the high-concentration carbon dioxide gas to obtain a methanol feed gas; and
a methanol synthesis process of reacting carbon dioxide and hydrogen contained in the methanol feed gas to produce methanol.

2. The method of synthesizing methanol according to claim 1, wherein water added to the feed gas in the water addition process is water produced as a by-product in the methanol synthesis process.

3. The method of synthesizing methanol according to claim 2, wherein the methanol synthesis process includes a membrane separation process of separating the water produced as a by-product of the reaction of the carbon dioxide and the hydrogen by passing the water through a membrane, and the water separated in the membrane separation process is used as water added to the feed gas in the water addition process.

4. The method of synthesizing methanol according to claim 3, wherein the membrane separation process is carried out in the same vessel as the methanol synthesis process.

5. The method of synthesizing methanol according to claim 3 or 4, wherein the membrane separation process includes a sweep process of passing the feed gas through one side of the membrane and sweeping water that has permeated the membrane with a sweep gas, with the feed gas being used as the sweep gas, and the feed gas to which water that has permeated the membrane is added being the hydrated feed gas.

6. The method of synthesizing methanol according to any one of claims 1 to 5, wherein a method used in the desulfurization process is a dry desulfurization method using a dry desulfurizing agent containing an iron compound selected from iron oxide and iron hydroxide.

7. The method of synthesizing methanol according to claim 6, wherein part or all of the iron compound is derived from a precipitate generated in water treatment at a steelworks.

8. The method of synthesizing methanol according to any one of claims 1 to 5, wherein a method used in the desulfurization process is a dry desulfurization method using a dry desulfurizing agent containing at least one compound of an element selected from zinc and copper.

9. The method of synthesizing methanol according to any one of claims 1 to 8, further comprising a heat exchange process of exchanging heat between the shift reaction gas and the hydrolyzed gas.

10. The method of synthesizing methanol according to any one of claims 5 to 9, further comprising a water washing process of bringing the feed gas into contact with liquid water and washing, before the sweep process.

11. The method of synthesizing methanol according to any one of claims 1 to 10, wherein a catalyst used in the hydrolysis process is a catalyst containing an oxide of Al.

12. The method of synthesizing methanol according to any one of claims 1 to 11, wherein a catalyst used in the water-gas shift reaction process is a catalyst containing a sulfide of at least one element selected from Co, Ni, and Mo.

13. The method of synthesizing methanol according to any one of claims 1 to 12, wherein a catalyst used in the water-gas shift reaction process is a catalyst containing an oxide of at least one element selected from Fe and Cr.

14. The method of synthesizing methanol according to any one of claims 1 to 13, further comprising a hydrogen gas separation process of separating hydrogen gas from gas of components other than the carbon dioxide separated in the carbon dioxide gas separation process.

15. The method of synthesizing methanol according to any one of claims 1 to 14, wherein the feed gas is an exhaust gas discharged from a reduction furnace.

16. The method of synthesizing methanol according to claim 15, wherein the reduction furnace is a blast furnace in a steelworks, and the feed gas is blast furnace gas.
